# EUROPEAN PATENT SPECIFICATION

(11) **EP 2 646 070 B1**
(45) Date of publication and mention of the grant of the patent: **28.01.2015**
(21) Application number: 11788165.6
(22) Date of filing: 28.11.2011
(51) Int. Cl.: A61M 5/14, A61M 5/142, A61B 5/151

(54) **MULTI-RESERVOIR PUMP PATCH**
PUMPENPFLASTER MIT MEHREREN RESERVOIRS
PATCH DE POMPE À RÉSERVOIRS MULTIPLES

(30) Priority: 29.11.2010 EP 10192989; 18.01.2011 US 201161433800 P
(43) Date of publication of application: 09.10.2013
(73) Proprietor: Sanofi-Aventis Deutschland GmbH, 65929 Frankfurt am Main (DE)
(72) Inventor: KOUYOUMJIAN, Garen, Leamington Spa Warwickshire CV31 1HN (GB); BOYD, Malcolm Stanley, Wellsbourne Warwickshire CV35 9PW (GB); DE SAUSMAREZ LINTELL, Daniel Thomas, Warwickshire CV23 9EQ (GB)
(74) Representative: Keil & Schaafhausen Patent- und Rechtsanwälte
(86) International application number: PCT/EP2011/071131
(87) International publication number: WO 2012/072555

(56) References cited:
- WO-A2-2007/108987
- US-A1- 2002 072 733
- US-A1- 2002 173 772
- US-A1- 2005 100 937

## Description

### Field of the Present Patent Application

The present application relates to medical devices delivering at least two drug agents from separate reservoirs or containers. More specifically, the present invention is concerned with patch-like, wearable, self-contained substance infusion devices that can be used to deliver medicaments to a patient through a single dispense interface. The drug agents may be available in two or more reservoirs, containers, or packages, each containing independent (single drug compound) or pre-mixed (co-formulated multiple drug compounds) drug agents.

### Background

Certain disease states require treatment using one or more different medicaments. Some drug compounds need to be delivered in a specific relationship with each other in order to deliver the optimum therapeutic dose. This invention is of particular benefit where combination therapy is desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus.

There are a number of potential problems when delivering two medicaments or active agents simultaneously. The two active agents may interact with each other during the long-term, shelf life storage of the formulation. Therefore, it is advantageous to store the active components separately and only combine them at the point of delivery, e.g. injection, needle-less injection, pumps, or inhalation. However, the process for combining the two agents needs to be simple and convenient for the user to perform reliably, repeatedly and safely.

A further problem is that the quantities and/or proportions of each active agent making up the combination therapy may need to be varied for each user or at different stages of their therapy. For example one or more actives may require a titration period to gradually introduce a patient up to a "maintenance" dose. A further example would be if one active requires a non-adjustable fixed dose while the other is varied in response to a patient's symptoms or physical condition. This problem means that pre-mixed formulations of multiple active agents may not be suitable as these pre-mixed formulations would have a fixed ratio of the active components, which could not be varied by the healthcare professional or user.

Additional problems arise where a multi-drug compound therapy is required, because many users cannot cope with having to use more that one drug delivery system or make the necessary accurate calculation of the required dose combination. This is especially true for users with dexterity or computational difficulties. In some circumstances it is also necessary to perform a priming procedure of the device and/or needle cannulae before dispensing the medicaments. Likewise, in some situations, it may be necessary to bypass one drug compound and to dispense only a single medicament from a separate reservoir.

WO 2007/108987 A2 discloses an infusion system according to the preamble of claim 1 including a first source having a reservoir which may be filled with a fast acting insulin and delivered using a pump to a first port and a cannula. Further, a second funnel port is provided for slow acting insulin which permits the slow acting insulin to be received from an instrument, such as a syringe, from external to the system. The infusion system further includes one-way valves, wherein the first valve is between the pump and the reservoir and the second valve is between the first port and the pump.

Document US 2002/0072733 A1 describes a device for delivering a fluid to a patient, including an exit port, a dispenser for causing fluid from a reservoir to flow to the exit port, a local processor programmed to cause a flow of fluid to the exit port based on flow instructions from a separate, remote control device, and a wireless receiver connected to the local processor for receiving the flow instructions.

### SUMMARY

Accordingly, there exists a strong need to provide devices and methods for the delivery of two or more medicaments in a single injection or delivery step that is simple for the user to perform. Our invention is defined in the appended claims and overcomes the above-mentioned problems by providing separate storage containers for two or more active drug agents that are then only combined and/or delivered to the patient during a single delivery procedure. Preferably, but not necessarily, setting a dose of one medicament through adjustment of a flow rate will automatically fix or determine the dose of the second medicament (i.e. non-user settable). Our invention also gives the opportunity for varying the quantity of one or both medicaments. For example, one fluid quantity can be varied by changing the properties of the injection device (e.g. allowing the device to accept a bolus of a secondary medicament into the flow of the primary medicament). The second fluid quantity can be changed by manufacturing a variety of secondary drug containing packages with each variant containing a different volume and/or concentration of the second active agent. The user or healthcare professional would then select the most appropriate secondary package or series or combination of series of different packages for a particular treatment regime. In other case not forming part of the invention, the patch pump device may be programmed remotely such that two separate reservoirs of medicament are pumped or infused through a single dispense interface at different rates.

We also disclose a medicated module that can be removably engaged with an external port on the exterior of the patch pump housing to automatically cause a secondary medicament to be dispensed into a manifold that is in fluid communication with the primary medicament and are infused into a user through a single dispense interface, preferably a needle placed subcutaneously. Such a configuration eliminates the need for the user to manually set or adjust a flow of secondary medicament from a second reservoir contained within the patch pump.

These and other advantages will become evident from the following more detailed description.

Our invention allows complex combinations of multiple drug compounds within a single drug delivery system. The invention can allow the user to set a single dispense rate that will result in the delivery of a multi-drug compound through a single dispense interface. This single dose rate setter controls the mechanism of the device such that a predefined combination of the individual drug compounds is delivered when the primary rate is set and medicament delivered through the single dispense interface.

More specifically, our invention relates to a patch pump designed to use an internal pump to dispense one or more medicaments directly to a user. The pump is intended to provide the user with a continuous dose of a primary drug via a dispense interface, such as a single needle cannula placed subcutaneously. The device also has the capability to dispense one or more secondary drugs via this same dispense interface. This provides flexibility of therapy where more than one medicament has been prescribed for the user or where a short-term additional medicament is required in combination with the longer term patch pump delivered medicament. A patch type pump is intended to form a self-contained unit containing the drive system, drug reservoir and dispense interface in a single device that may or may not also feature controls for operating the device either integrated or remotely. Preferably, the patch pump is designed to be worn or removably attached to a user's skin.

Examples of patch pump type devices that can incorporate our invention are described in issued patent US 6,749,587. The '587 patent describes the structure and functionality of drive mechanisms, controllers, propulsion means, flow paths, needle assembles, and housing designs for pump patch type injection devices, specifically the interaction of electronic circuits, propulsion means and medicament reservoir during both dose setting and dose delivery.

Although the patch pump embodiments of our invention are similar to those used in the treatment of diabetes whereby the medicaments are infused as liquids subcutaneously through a needle and are left on the body whether controlled remotely or not, the principles of our invention could be employed by similar devices not forming part of the invention where another format of drug (gas, powder etc.) is supplied over time to a user or patient (intravenous, respiratory, oral etc.) through any form of shared dispense interface (needle, mask, tracheal tube etc.). Importantly, the secondary medicament as described in the embodiments herein need not be a different compound to the primary drug, for example, an insulin bolus (secondary) added to basal insulin flow (primary). It may even be appropriate that the second compound be a diluent or carrier compound for the primary drug. The use of such a carrier e.g. saline solution, may temporarily alter the combined therapeutic profile or therapeutic benefit where a deviation is required for a short period.

Each of the embodiments of the present invention require at least two separate reservoirs, where at least one reservoir is contained within the pump patch housing and is designed to dispense a primary medicament through a manifold and out a single dispense interface, preferably a cannula that protrudes through the housing of the patch pump. In embodiments not forming part of the invention the additional reservoirs can be inside the housing or removably attached to a port on the housing, as in the case of the medicated module embodiment. In the configurations where the secondary reservoir(s) are contained in the housing, the reservoir(s) could be pre-filled before delivery to the user (to improve usability or minimize filling errors) or filled by the user in the same manner as the primary reservoir in most current, conventional insulin pumps. In this case, the secondary drug reservoir can either be flushed by the flow of the primary drug (in-line placement) or actuated independently from the primary reservoir (parallel dispense) and combined with the primary drug during dispense to the user through the single dispense interface.

According to the invention, the additional reservoir is inside the housing removably attached to a port, e.g. a loading port in the housing. The additional or second reservoir has an inlet and an outlet and is replaceable through a loading port in the housing, e.g. a pump patch housing, where the second reservoir is connected to a manifold such that primary medicament flowing from the first reservoir flows in the inlet and out the outlet of the secondary reservoir.

Alternatively, but not forming part of the invention, the secondary reservoir can be housed in an external device, e.g., a medicated module that is introduced to the primary device at such a time as the secondary drug is required. As used herein, the medicated module includes any type of standalone device comprising housing containing a cavity or reservoir of a single dose of medicament, where the housing is attachable to an external port on a patch pump. Through activation of medicated module when attached to the patch pump, the secondary medicament is caused to flow into the manifold of the patch pump and eventually delivered to a user through a single dispense interface, preferably a needle cannula. Again, this introduction can be in-line with the primary medicament or parallel and combined before dispense.

One embodiment for such an external device could be a modified single-use drug reservoir needle having a safety needle guard. Using such a device, the actuation of the needle guard being pressed against the patch pump port can drive the secondary medicament into the patch pump manifold system as and when the additional/secondary medicament is required. This system could be used where various secondary medicaments are required at varying time intervals compared with the constant primary medicament flow. The user or physician could then add the additional medicament(s) as and when required, using it as a form of injection port for additional injections, thereby reducing the discomfort and risk of infection associated with additional skin punctures.

In the case of in-line introduction of the secondary medicaments according to the invention, the secondary medicament is supplied as a capsule that is placed in series with delivery of the primary medicament, the delivery of which pushes or flushes this secondary medicament through (i.e., a single drive mechanism delivers both medicaments). The secondary medicament reservoir comprises an inlet and an outlet and is replaceable through a loading port in the housing, e.g. a housing of a patch pump device. The secondary medicament reservoir is connected to a manifold such that primary medicament flowing from the first reservoir flows in the inlet and out the outlet of the secondary reservoir. In an additional embodiment, a bypass route may be incorporated into the device design to allow flow of only the primary drug to the user when the secondary drug is not required. The drug flow can then be rerouted via the secondary reservoir as and when needed by a linearly or rotationally actuated section of the flow channel. A simple system of one-way valves can be used to prevent back-flow, accidental mixing and leaking from the system.

A drug delivery device according to the invention may further comprise a movable inner housing having two flow path positions. In one position, a first position, the second reservoir is placed in-line with the first reservoir. In another position, a second position, the second reservoir is placed out of line with the first reservoir. The housing may be slidable or rotatable. The drug delivery device may comprise a rotatable inner housing, wherein the inner housing may rotate about an axis to place the second reservoir in-line with a manifold such that the primary medicament will dispense, flush, or drive the secondary medicament from the second reservoir. The drug delivery device may comprise an inner housing further comprising a flow path. When the inner housing is in the second position the flow path may be placed in-line with manifold. This provides a benefit of allowing use of the drug delivery device in situations where delivery of the secondary medicament is not needed. However, the device may be configured to allow repositioning of the inner housing between the first position and the second position. The drug delivery device may comprise an opening or loading port in or on the housing and a movable inner housing. Positioning of the inner housing into the second position may allow for the replacement of second reservoir through the opening (not shown) in the housing of the drug delivery device. This provides the benefit of allowing replacement of the secondary reservoir while the medicament from the first reservoir is delivered.

In another embodiment relating to parallel dispense, not forming part of the invention, the patch pump device can be supplied with several additional secondary medicaments loaded such that the user may decide when these are needed and dispense them into the manifold and flow of the primary medicament as required. As with other embodiments of this nature, a system of one-way valves can be used to prevent back-flow, accidental mixing and leaking from the system to permit efficient delivery of combined medicaments via the same dispense interface.

By defining the therapeutic relationship between the individual drug compounds our delivery device would help ensure that a patient/user receives the optimum therapeutic combination dose from a multi-drug compound device without the inherent risks associated with multiple inputs where the user has to calculate and set the correct dose combination every time they use the device. The medicaments can be fluids, defined herein as liquids, gases or powders that are capable of flowing and that change shape at a steady rate when acted upon by a force tending to change its shape. Alternatively, one of the medicaments may be a solid that is carried, solubilized or otherwise dispensed with another fluid medicament or carrier.

According to one specific aspect this invention is of particular benefit to users with dexterity or computational difficulties as the single input and associated predefined therapeutic profile removes the need for them to calculate their prescribed dose every time they use the device and the single input allows considerably easier setting and dispensing of the combined compounds.

In a preferred embodiment a master or primary drug compound, such as insulin, is contained within a multiple dose first reservoir connected to a miniaturized pump mechanism known to those skilled in the art of infusion devices that is capable of delivering a continuous dose of medicament and/or a pulse (bolus) of medicament. The pump mechanism preferably is battery operated and controlled through one or more electronic circuits that are manually and/or remotely operated and/or programmed. The pump mechanism is in fluid communication with a manifold and contained within a housing having a bottom surface configured to be removably attached to a user's skin, for example through an adhesive. Also in fluid communication with the manifold is a cannula having distal and proximal ends, where the distal end protrudes through the housing of the patch pump, preferably through an opening in the bottom surface. A preferred cannula is one where the distal end is sharpened like the distal end of an injection needle. Alternatively, the cannula may be of a flexible material for improved user comfort and may or may not be inserted by a separate needle (similar to an injection needle) that is retracted by the device after insertion of the cannula.

The first reservoir can be any type of container, such as a cartridge, and can be filled with primary medicament prior to delivery to the user or it can be filled by the user (or health care provider) immediately prior to use by using an external source, such as, a standard syringe through a port in the housing. The first reservoir can be re-useable, disposable, or non-replaceable. The second reservoir, when contained within the housing can be of the same design as the first reservoir, preferably a replaceable container, most preferably containing a single dose of a secondary medicament.

Although our invention specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with our invention.

For the purposes of our invention the term "insulin" shall mean Insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyldes(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH₂), Exendin-3, Liraglutide, or AVE0010 (H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-Lys-Lys-Lys-Lys-Lys-Lys-NH₂).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

In one embodiment there is provided a medicated module attachable to the patch pump drug delivery device that comprises an outer housing having a proximal end, a distal end, and an outer surface. There is a reservoir in the housing that contains a secondary medicament. The medicated module assembly contains a needle guard that can reduce the risk of accidental needle sticks before and after use as well as preventing a user from using the module a subsequent time when the secondary medicament has already been expelled. The movable needle guard or shield is configured to move axially in both the distal and proximal directions when pressed against and removed from a port in the patch pump housing. When the needle assembly is removed from the port, the guard is returned to post-use extended position to securely lock the guard from further substantial axial movement. By "substantial" movement we do not mean the typical amount of "play" in a system, but instead we mean that the guard and/or distal needle do not move axially a distance that exposes the distal end of the cannula once it is locked out.

Inside the medicated module housing there is a cavity that contains a capsule, which comprises a single dose of medicament in a reservoir. As the needle guard is retracted during an application to the port on the patch pump housing, a trigger button on the device is unlocked. Activation of this trigger button releases some form of stored internal energy (such as a compressed spring, pressurized gas or electric battery) that acts to force the capsule onto the distal cannula of the module and further to compress the capsule, thus decreasing its volume and dispensing its contents. This allows the seals of the capsule to be pierced by the needle cannula such that the medicament can be expelled from the reservoir and into the manifold within the patch pump housing. The medicated module is preferably designed in such a way as to limit its use to one exclusive patch pump device (or family of devices) through employment of dedicated/coded/exclusive features to prevent attachment of a non-appropriate medicated module to a non-matching device.

A particular benefit is that the medicated module makes it possible to tailor dose regimes when required, especially where a titration period is necessary for a particular drug. The medicated module could be supplied in a number of titration levels with obvious differentiation features such as, but not limited to, aesthetic design of features or graphics, numbering etc, so that a patient could be instructed to use the supplied medicated module in a specific order to facilitate titration. Alternatively, the prescribing physician may provide the patient with a number of "level one" titration medicated modules and then when these were finished, the physician could then prescribe the next level. A key advantage of this titration program is that the primary device remains constant throughout.

Should the patient attempt to reuse a previously used medicated module, our invention includes the locking needle guard that is activated after a first predefined travel/retraction of the guard/insertion of the needle. The locked needle guard would alert the patient to this situation and the inability to use the module for a second time. Visual warnings (e.g. change in color and/or warning text/indicia within an indication window on the module once insertion and/or fluid flow has occurred) can also be used. Additionally, tactile feedback (presence or absence of tactile features on the outer surface of the module hub following use) could be used as well.

These as well as other advantages of various aspects of the present invention will become apparent to those of ordinary skill in the art by reading the following detailed description, with appropriate reference to the accompanying drawings.

### BRIEF DESCRIPTION OF THE DRAWINGS

Exemplary embodiments are described herein with reference to the drawings, in which:
Figure 1 illustrates a sectional view of one possible drug delivery device containing a first and second reservoir;
Figure 2 illustrates a sectional view of another embodiment with multiple second reservoirs;
Figure 3 illustrates a schematic representation of another embodiment where the reservoirs can be filled by external means, such as a syringe;
Figure 4 illustrates a schematic representation of another embodiment where the reservoirs are in an in-line flow configuration;
Figure 5 illustrates a schematic representation of an embodiment of our invention where the reservoir of secondary medicament is modular and replaceable in an in-line flow configuration;
Figure 6 illustrates a schematic representation of yet another embodiment where the second reservoir is part of an inner housing that can be moved into the flow path of the primary medicament;
Figure 7 illustrates a perspective sectional view of another embodiment where the second reservoir is part of a medicated module attachable to the device housing; and
Figure 8 illustrates a schematic representation of another embodiment where multiple medicated modules are attached to the housing of the device.

### DETAILED DESCRIPTION

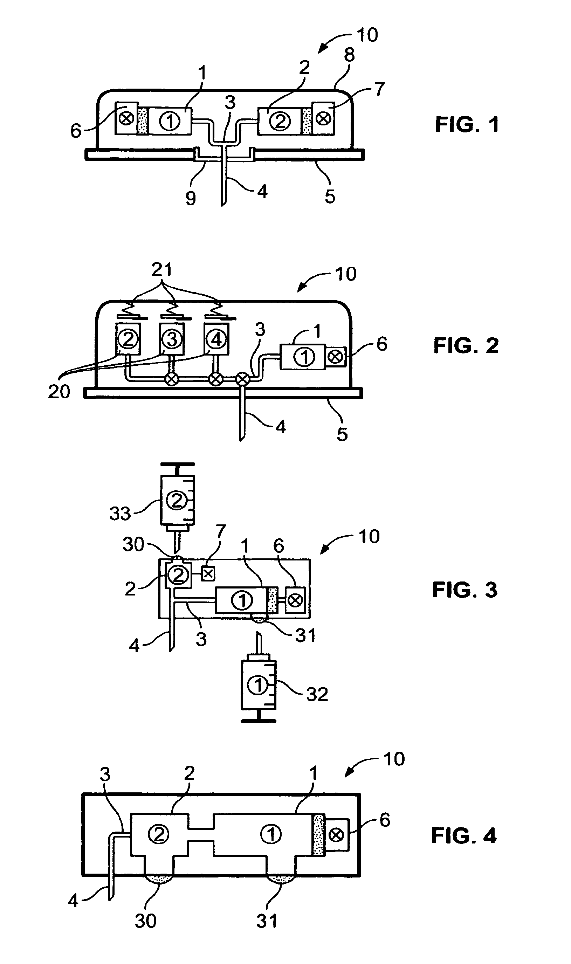

FIGS. 1 through 8 illustrate exemplary embodiments of a fluid delivery device 10. The types of liquids that can be delivered by the fluid delivery device of the present invention include, but are not limited to, insulin, antibiotics, nutritional fluids, total parenteral nutrition or TPN, analgesics, morphine, hormones or hormonal drugs, gene therapy drugs, anticoagulants, analgesics, cardiovascular medications, AZT or chemotherapeutics. The types of medical conditions that the fluid delivery device of the present invention might be used to treat include, but are not limited to, diabetes, cardiovascular disease, pain, chronic pain, cancer, neurological diseases, Alzheimer's Disease, ALS, Hepatitis, Parkinson's Disease or spasticity.

Referring first to FIG. 1, the present disclosure provides a fluid delivery device 10, wherein only certain of the components of the device 10 are shown, namely reservoir 1 for storage of a primary medicament, reservoir 2 for storing a secondary medicament, manifold 3 in fluid communication with both reservoirs and with single dispense interface or cannula 4. Electronic circuits, controllers, processors, power sources, and the like are not illustrated for the sake of brevity and clarity. Propulsion or pump mechanisms 6 and 7 provide the driving force to cause each medicament to flow from reservoirs 1 and 2, respectively, into manifold 3 and out the distal end of cannula 4. Each pump mechanism can be independently or dependently controlled such that the relative flow or amount of the primary and secondary medicament can be varied to correspond to a predetermined titration profile.

The device 10 has a housing 8 with a bottom surface 5. The device can comprise two or more assemblies that are assembled together for use, and wherein one of the assemblies is disposable and the other of the assemblies is reusable. The reusable assembly can be removably attachable to the disposable assembly, for example, where the reusable assembly may be designed to include the more costly components of fluid delivery device 10. Preferably the fluid delivery device 10, with assemblies attached, is small, with a cross-sectional area about the size of a credit card and a thickness less than one inch to facilitate wearability by the user. The bottom surface 5 of housing 8 preferably includes an exit port assembly 9 including a transcutaneous patient access tool, such as the rigid or flexible cannula 4 for penetrating the skin of a patient.

Although not shown, a processor or electronic microcontroller is connected to a power source and the propulsion means and can include a microprocessor, an electronic memory, an electronic clock oscillator, an analog to digital converter and a multiplexer.

The processor is programmed to cause a flow of one or more medicaments through manifold 3 to the exit port assembly 9 based on flow instructions from a separate, remote control device (not shown). The fluid delivery device 10 preferably further includes a wireless receiver connected to the processor for receiving the flow instructions from the separate, remote control device and delivering the flow instructions to the processor. Alternatively, the device could have a number of externally accessible user input features to allow the user or a health care provider to program the device.

Control of fluid flow can be through the use of actuators such as solenoids, piezo actuators, magnetic actuators, thermal generators or other mechanisms to generate, direct or redirect a force, temperature gradient, electromagnetic field or other medium for controlling flow of one or more medicaments between the reservoirs and the exit port assembly. If the reservoirs are maintained at ambient pressure, metering of the flow can include a rotary peristaltic head, linear peristaltic mechanism, electromagnetic fluid propulsion, a displacement pump, or other means for moving fluid from the reservoirs to the exit port assembly. A rotary peristaltic head that is driven by a motor drive may accomplish such propulsion means. If the reservoir is pressurized, one or more valves may simply control flow, and not to propel the fluid. The reservoirs may be included in a sealed compartment, and pressurized gas provide the driving force, or the reservoirs may be in contact with a force generating member such as a spring, a separate elastomeric structure, or a cantilever beam attached to the housing of the device.

As shown in the figures, housing 8 is free of user input components, such as electromechanical switches or buttons or interfaces otherwise accessible to a user to adjust the programmed flow rate through the processor, for providing flow instructions to the device. The lack of user input components allows the size, complexity and costs of the device 10 to be substantially reduced so that the device 10 lends itself to being small and either partially or fully disposable in nature. The bottom surface 5 of housing 8 can contain an adhesive layer (not shown) comprising a standard biocompatible glue such as those used in common adhesive bandages, and may include a protective covering to avoid the adhesive sticking to unwanted objects prior to attachment to the skin of the patient. The skin penetrating cannula 4 can be introduced through the patient's skin prior to attachment of the device to the patient's skin, at the same time as it is attached to the patient's skin, or after it is attached to the patient's skin. Simultaneous puncturing of the patient's skin by the penetrating cannula 4 and attachment of the device may be preferred to simplify attachment and reduce pain of transcutaneous puncturing. The sub-assembly comprising the cannula and adhesive patch may be designed to be detachable from the housing 8 so as to permit reusability of the main body of the device while allowing for disposal of components in contact with the patient.

FIG. 2 illustrates another embodiment where the primary medicament is contained in reservoir 1 and expelled into manifold 3 by pump mechanism 6, however, instead of only a single reservoir of secondary medicament, the device 10 can be configured to have one or more reservoirs 20 to hold one or more secondary medicaments. These additional reservoirs are in parallel flow with the primary medicament from reservoir 1 and each is in fluid communication with manifold 3 and cannula 4. Additional reservoirs 20 can each have an independently controlled propulsion mechanism 21 that can cause the secondary medicament to be pulsed into the manifold or introduced continuously at a predetermined flow rate.

FIG. 3 shows another embodiment where reservoirs 1 and 2 are reusable and can be filled from an external source, such as syringes 32 and 33, through ports 31 and 30, respectively. These ports preferably comprise a septum that can be easily pierced by the syringe needle. In this embodiment the port 31 for filling the primary drug reservoir 1 is located on the underside of the device such that reservoir 1 cannot be filled once the device has been attached to the user's skin, thereby preventing overloading the device or forcing an overdose of the primary medicament. Port 30 can be used to fill reservoir 2 periodically whenever it is necessary to administer the secondary medicament.

FIG. 4 shows a similar embodiment as shown in FIG.3 except reservoir 2 is an in-line flow configuration with reservoir 1 as opposed to the parallel flow configuration shown in FIG.3 and features both reservoir filling ports 30 and 31 located on the underside of the device to prevent filling of either reservoir once the device has been attached to the user. Using an in-line flow configuration can eliminate the need for a separate propulsion mechanism for reservoir 2 because the primary medicament from reservoir 1 provides the driving force to push (flush) the secondary medicament from reservoir 2 into manifold 3.

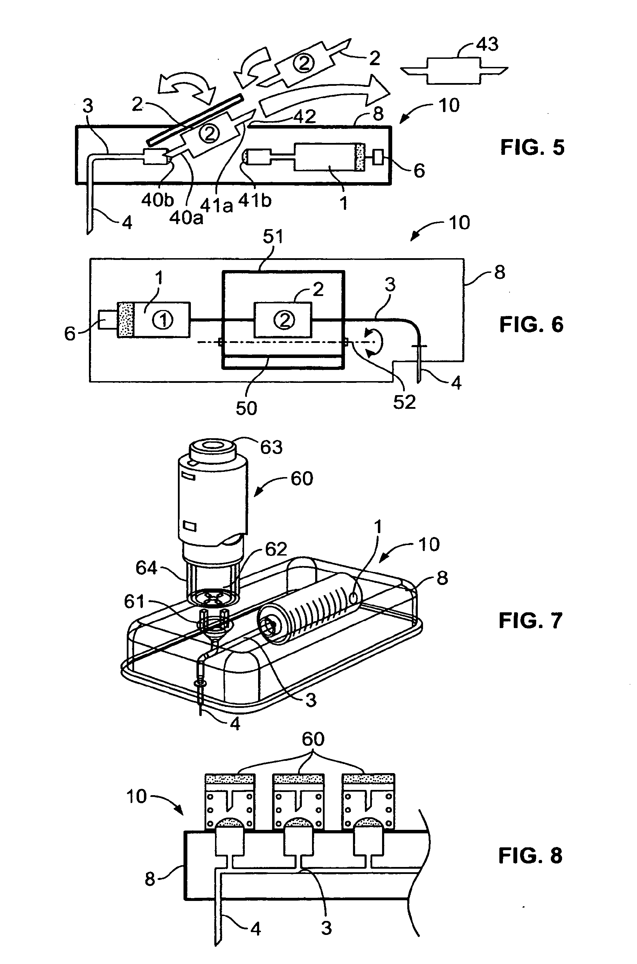

FIG. 5 is another variation of the in-line flow configuration of FIG. 4. Here reservoir 2 is designed to be a modular component that can be replaced through an opening 42 in housing 8. Reservoir 2 can have connectors 40a and 41 a, such as needle cannula, that fluidly connect with manifold connectors 40b and 41 b, preferably pierceable septa. The device of FIG. 5 can also comprise a "blank" reservoir 43 that can be inserted into manifold 3 to complete the flow path, but not contain any secondary medicament. FIG. 6 is yet another variation of the in-line flow configuration where a rotatable or sliding inner housing 51 has two flow path positions; one with reservoir 2 inline and a second without a reservoir. FIG.6 illustrates the configuration where inner housing 51 has been rotated about axis 52 to place reservoir 2 in-line with manifold 3 such that the primary medicament will dispense, flush, or drive the secondary medicament from reservoir 2. In situations where delivery of the secondary medicament is not needed, the inner housing can be repositioned such that flow path 50 is placed in-line with manifold 3. Repositioning of the inner housing can also allow for the replacement of new reservoir 2 through a suitable opening (not shown) in housing 8.

Each of the above described embodiments require that all the reservoirs are located within housing 8 during dispense. FIGS. 7 and 8 illustrate embodiments where one or more reservoirs containing secondary medicament(s) are external to device 10. FIG. 7 shows a configuration where a medicated module 60 contains a reservoir 62 of secondary medicament and is removably attachable to port 61 in housing 8 of device 10. Each medicated module is preferably self-contained and provided as a sealed and sterile disposable module that has an attachment means compatible to the attachment means on housing 8 of device 10. Although not shown, the medicated module could be supplied by a manufacturer in a protective and sterile container, where the user would peel or rip open a seal or the container itself to gain access to the sterile medicated module. In some instances it might be desirable to provide two or more seals for each end of the medicated module.

Once module 60 is connected to port 61 the user can activate module 60 by pressing button or trigger 63 to cause the secondary medicament to be dispensed into manifold 3 where it is carried out through cannula 4 by the flow of the primary medicament from reservoir 1. This secondary compound can either be dispensed into a live flow such that the module would remain attached until the dose was delivered or, it could dispense into the manifold 3 and be gradually dispensed into the user by the flow of primary medicament. The module 60 and port 61 can have matching dedicated connectors to prevent the module from connecting to a non-authorized device. Any known attachment means can be used to attach the medicated module to device 10, including all types of permanent and removable connection means, such as threads, snap locks, snap fits, luer locks, bayonet, snap rings, keyed slots, and combinations of such connections. A guard 64 can also be used to protect the dispense interface of the module and/or the user prior to attachment to device 10. Once the secondary medicament is dispensed and the module is disconnected from port 61, the guard 64 will lock into place and prevent re-attachment to the device. FIG. 8 shows a further embodiment of the configuration of FIG. 7 where multiple modules 6 can be attached (removably or permanently) to device 10 housing 8. Each of the modules can be activated independently of the others. In both embodiments the secondary medicament can be introduced to the device multiple times (with multiple medicated modules) without removing the device 10 from the user's body. This allows for the introduction of medicament subcutaneously by sharing the cannula 4 already inserted in the user by the device, thereby reducing the number of required injection sites and subsequent discomfort and risk of infection.

Materials of construction for the medicated module and device 10 include, but are not limited to, COC (an amorphous polymer based on ethylene and norbonene, also referred to as cyclic olefin copolymer, ethylene copolymer, cyclic olefin polymer, or ethylene-norbornene copolymer); LCP (a liquid crystal polymer having an aramid chemical structure that includes linearly substituted aromatic rings linked by amide groups, and further can include partially crystalline aromatic polyesters based on p-hydroxybenzoic acid and related monomers and also highly aromatic polyesters); PBT (polybutylene terephthalate thermoplastic crystalline polymer or polyester); COP (a cyclic olefin polymer based on ring-opening polymerization of norbornene or norbornene-derivatives); HDPE (high density polyethylene); and SMMA (styrene methyl methacrylate copolymer based on methyl methacrylate and styrene). The needle pierceable septa, bungs, and/or seals that are used with both the module and device 10 can be manufactured using TPE (thermo plastic elastomer); LSR (liquid silicone rubber); LDPE (low density polyethylene); and/or any kind of medical grade rubber, natural or synthetic.

Exemplary embodiments have been described. Those skilled in the art will understand, however, that changes and modifications may be made without departing from the scope of the present invention, which is defined by the claims.

### List of References

- 1: reservoir
- 2: reservoir
- 3: manifold
- 4: single dispense interface/cannula
- 5: bottom surface
- 6: propulsion/pump mechanism
- 7: propulsion/pump mechanism
- 8: housing
- 9: exit port assembly
- 10: device
- 20: reservoir
- 21: propulsion/pump mechanism
- 30: port
- 31: port
- 32: syringe
- 33: syringe
- 40a: connector
- 40b: manifold connector
- 41a: connector
- 41b: manifold connector
- 42: housing opening
- 43: blank reservoir
- 50: flow path
- 52: axis
- 60: medicated module
- 61: port
- 62: reservoir
- 63: button/trigger
- 64: guard

## Claims

1. A drug delivery device comprising,
a. a housing (8) having a bottom surface (5) configured to removably attach directly to a user's skin;
b. a medicament manifold (3) contained within the housing;
c. a single dispense interface (4) having a distal end and a proximal end, where the proximal end is fluidly connected to the manifold and the distal end protrudes through the housing and is configured for subcutaneous placement in a user' skin;
d. a first reservoir (1) and a second reservoir (2) each connected to the manifold; and
e. a pump connected (6) to the first reservoir configured to continuously dispense a primary medicament contained in the first reservoir through the manifold into the proximal end of the single dispense interface and then out the distal end **characterized in that**
the second reservoir (2) has an inlet (41a) and an outlet (40a) and is replaceable through a loading port (42) in the housing, where the second reservoir is connected to the manifold such that primary medicament flowing from the first reservoir (1) flows in the inlet and out the outlet of the second reservoir.

2. The device of claim 1 where the single dispense interface comprises a needle cannula (4).

3. The device of any preceding claim where the second reservoir (2) comprises at least one dose.

4. The device of any preceding claim where the second reservoir (2) contains a filling port (30) configured to allow filling of the second reservoir with a secondary medicament from an external source (33) of secondary medicament located outside of the housing.

5. The device of any preceding claim where the distal end of the single dispense interface comprises a cannula (4) that protrudes through the bottom surface (5) of the housing (8) and is configured to dispense the primary and secondary medicaments subcutaneously.

6. The device of claim 1, further comprising a movable (rotatable or sliding) inner housing having two flow path positions; wherein in a first position the second reservoir is placed in-line with the first reservoir and in a second position the second reservoir is placed out of line with the first reservoir.

7. The device of claim 6, wherein the inner housing (51) is rotatable about axis (52) to place the second reservoir (2) in-line with the manifold (3) such that the primary medicament dispenses, flushes, or drives the secondary medicament from the second reservoir.

8. The device of any of claims 6 or 7, wherein the inner housing (51) further comprise a flow path (50), wherein when the inner housing (51) is in the second position, flow path (50) is placed in-line with manifold (3).

9. The device of any of claims 6 to 8 wherein repositioning of the inner housing (51) into the second position allows for the replacement of reservoir (2) through an opening (not shown) in the housing (8).

## Patentansprüche

1. Arzneimittelabgabevorrichtung, umfassend:
a. ein Gehäuse (8) mit einer Unterseite (5), die zur direkten lösbaren Befestigung an der Haut eines Anwenders ausgelegt ist;
b. einen Medikamentenverteiler (3), der in dem Gehäuse enthalten ist;
c. eine einzelne Abgabeschnittstelle (4) mit einem distalen Ende und einem proximalen Ende, wobei das proximale Ende mit dem Verteiler in Fluidverbindung steht und das distale Ende durch das Gehäuse vorsteht und zur subkutanen Platzierung in der Haut eines Anwenders ausgelegt ist;
d. ein erstes Reservoir (1) und ein zweites Reservoir (2), die jeweils an dem Verteiler angebracht sind; und
e. eine an dem ersten Reservoir angebrachte Pumpe (6) zur kontinuierlichen Abgabe eines in dem ersten Reservoir enthaltenen primären Medikaments durch den Verteiler in das proximale Ende der einzelnen Abgabeschnittstelle und aus dem distalen Ende,
**dadurch gekennzeichnet, dass**
das zweite Reservoir (2) einen Einlass (41a) und einen Auslass (40a) aufweist und durch eine Ladeöffnung (42) in dem Gehäuse ausgewechselt werden kann, wobei das zweite Reservoir mit dem Verteiler verbunden ist, so dass von dem ersten Reservoir (1) fließendes primäres Medikament in den Einlass und aus dem Auslass des zweiten Reservoirs fließt.

2. Vorrichtung nach Anspruch 1, worin die einzelne Abgabeschnittstelle eine Nadelkanüle (4) umfasst.

3. Vorrichtung nach einem der vorhergehenden Ansprüche, worin das zweite Reservoir (2) zumindest eine Dosis umfasst.

4. Vorrichtung nach einem der vorhergehenden Ansprüche, worin das zweite Reservoir (2) eine Füllöffnung (30) enthält, die ausgelegt ist, das Füllen des zweiten Reservoirs mit einem sekundären Medikament von einer externen Quelle (33) des sekundären Medikaments, die sich außerhalb des Gehäuses befindet, zu gestatten.

5. Vorrichtung nach einem der vorhergehenden Ansprüche, worin das distale Ende der einzelnen Abgabeschnittstelle eine Kanüle (4) umfasst, die durch die Unterseite (5) des Gehäuses (8) vorsteht und zur subkutanen Abgabe der primären und sekundären Medikamente ausgelegt ist.

6. Vorrichtung nach Anspruch 1, ferner ein bewegliches (drehbares oder gleitendes) Innengehäuse mit zwei Strömungswegstellungen umfassend; worin das zweite Reservoir in einer ersten Stellung in einer Linie mit dem ersten Reservoir platziert ist und das zweite Reservoir in einer zweiten Stellung nicht in einer Linie mit dem ersten Reservoir platziert ist.

7. Vorrichtung nach Anspruch 6, worin das Innengehäuse (51) um eine Achse (52) gedreht werden kann, um das zweite Reservoir (2) in einer Linie mit dem Verteiler (3) zu platzieren, so dass das primäre Medikament das sekundäre Medikament aus dem zweiten Reservoir abgibt, spült oder treibt.

8. Vorrichtung nach einem der Ansprüche 6 oder 7, worin das Innengehäuse (51) ferner einen Strömungsweg (50) umfasst, worin der Strömungsweg (50) in einer Linie mit dem Verteiler (3) platziert wird, wenn sich das Innengehäuse (51) in der zweiten Stellung befindet.

9. Vorrichtung nach einem der Ansprüche 6 bis 8, worin Umsetzen des Innengehäuses (51) in die zweite Stellung Auswechseln des Reservoirs (2) durch eine (nicht gezeigte) Öffnung in dem Gehäuse (8) gestattet.

## Revendications

1. Dispositif d'administration de médicament comprenant :
a. un boîtier (8) comportant une surface inférieure (5) configurée pour se fixer directement, de manière amovible, à la peau d'un utilisateur ;
b. une tubulure pour substance médicamenteuse (3) située à l'intérieur du boîtier ;
c. une interface de distribution unique (4) comportant une extrémité distale et une extrémité proximale, l'extrémité proximale étant raccordée en communication fluidique à la tubulure et l'extrémité distale faisant saillie à travers le boîtier et étant configurée pour être placée en position sous-cutanée sous la peau d'un utilisateur ;
d. un premier réservoir (1) et un second réservoir (2) raccordés chacun à la tubulure ; et
e. une pompe (6) raccordée au premier réservoir et configurée pour distribuer de façon continue une substance médicamenteuse principale située dans le premier réservoir par le biais de la tubulure de sorte qu'elle entre par l'extrémité proximale de l'interface de distribution unique puis sorte par l'extrémité distale
**caractérisé en ce que**
le second réservoir (2) comporte une entrée (41a) et une sortie (40a) et est remplaçable à travers un orifice de chargement (42) dans le boîtier, le second réservoir étant raccordé à la tubulure de telle sorte que la substance médicamenteuse principale s'écoulant depuis le premier réservoir (1) s'écoule de sorte qu'elle entre par l'entrée et sorte par la sortie du second réservoir.

2. Dispositif selon la revendication 1, dans lequel l'interface de distribution unique comprend une canule d'injection (4).

3. Dispositif selon l'une quelconque des revendications précédentes, dans lequel le second réservoir (2) comprend au moins une dose.

4. Dispositif selon l'une quelconque des revendications précédentes, dans lequel le second réservoir (2) contient un orifice de remplissage (30) configuré pour permettre le remplissage du second réservoir avec une substance médicamenteuse secondaire à partir d'une source extérieure (33) de substance médicamenteuse secondaire se trouvant à l'extérieur du boîtier.

5. Dispositif selon l'une quelconque des revendications précédentes, dans lequel l'extrémité distale de l'interface de distribution unique comprend une canule (4) qui fait saillie à travers la surface inférieure (5) du boîtier (8) et est configurée pour effectuer une distribution sous-cutanée des substances médicamenteuses principale et secondaire.

6. Dispositif selon la revendication 1, comprenant en outre un boîtier intérieur mobile (rotatif ou coulissant) ayant deux positions de trajet d'écoulement ; dans lequel, dans une première position, le second réservoir est placé de façon alignée avec le premier réservoir et, dans une seconde position, le second réservoir est placé de façon non alignée avec le premier réservoir.

7. Dispositif selon la revendication 6, dans lequel le boîtier intérieur (51) est rotatif autour de l'axe (52) en vue du positionnement du second réservoir (2) de façon alignée avec la tubulure (3) de telle sorte que la substance médicamenteuse principale distribue, évacue ou entraîne la substance médicamenteuse secondaire à partir du second réservoir.

8. Dispositif selon l'une quelconque des revendications 6 et 7, dans lequel le boîtier intérieur (51) comprend en outre un trajet d'écoulement (50), le trajet d'écoulement (50) étant, lorsque le boîtier intérieur (51) se trouve dans la seconde position, placé de façon alignée avec la tubulure (3).

9. Dispositif selon l'une quelconque des revendications 6 à 8, dans lequel le repositionnement du boîtier intérieur (51) dans la seconde position permet le remplacement du réservoir (2) à travers une ouverture (non illustrée) dans le boîtier (8).
